# EUROPEAN PATENT APPLICATION

(11) **EP 4 796 541 A1**
(43) Date of publication of application: **26.08.2026**
(21) Application number: 24878633.7
(22) Date of filing: 09.08.2024
(51) Int. Cl.: C07D 471/04, A61P 9/04, A61K 31/4375, A61P 13/12, A61P 7/12

(54) **FINERENONE INTERMEDIATE, PREPARATION METHOD THEREFOR AND PREPARATION METHOD FOR FINERENONE**

(30) Priority: 20.10.2023 CN 202311362850
(71) Applicant: SHANDONG CHENGCHUANG BLUE SEA PHARMACEUTICAL TECHNOLOGY CO., LTD., Jinan, Shandong 250101 (CN)
(72) Inventor: PENG, Xianglong, Jinan, Shandong 250101 (CN); LIU, Binbin, Jinan, Shandong 250101 (CN); CAO, Yan, Jinan, Shandong 250101 (CN); ZHOU, Xuyang, Jinan, Shandong 250101 (CN); LV, Zhitao, Jinan, Shandong 250101 (CN); YAO, Songzhi, Jinan, Shandong 250101 (CN); LI, Xiaowen, Jinan, Shandong 250101 (CN)
(74) Representative: Metida
(86) International application number: PCT/CN2024/111018
(87) International publication number: WO 2025/081981

(57) **Abstract**

The present invention belongs to the technical field of pharmaceutical synthesis, and specifically relates to a finerenone intermediate compound IV or compound V, preparation methods thereof, and a preparation method of finerenone.

## Description

### TECHNICAL FIELD

The present invention belongs to the technical field of pharmaceutical synthesis, and specifically relates to a finerenone intermediate compound V, a preparation method thereof, and a preparation method of finerenone.

### BACKGROUND

Finerenone, with the chemical name (4S)-4-(4-cyano-2-methoxyphenyl)-5-ethoxy-2,8-dimethyl-1,4-dihydro-1,6-naphthyri dine-3-carboxamide, is a non-steroidal mineralocorticoid receptor antagonist developed by Bayer AG. Finerenone is the first novel mineralocorticoid receptor antagonist approved for treating chronic kidney disease associated with diabetes. This product was approved in China in late June 2022. Since 2021, finerenone has been approved for marketing in multiple countries and regions including the United States and the European Union. Finerenone was approved in China within four months after its approval in the European Union, achieving near-global synchronization. This timely approval better addresses the urgent clinical need for novel therapeutics for diabetes-related chronic kidney disease in China. The structural formula of finerenone is as follows:

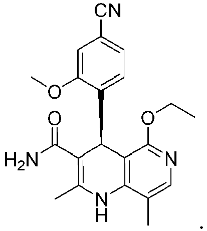

Multiple literature sources report preparation methods for finerenone.

CN106795155A discloses isolation of finerenone enantiomers from a racemate via chiral high-performance liquid chromatography (HPLC). Although finerenone prepared by this method exhibits high optical purity, the total yield is only 34.3%, which is relatively low. This low-yield process employs expensive simulated moving bed (SMB) equipment (Chiralpak AS-V, 20 µm column) for chiral resolution. Under Good Manufacturing Practice (GMP) conditions, the procurement cost for this equipment is high; currently, the industrial-scale equipment costs from several million to tens of millions of Chinese Yuan (CNY). Additionally, the process uses chromatographic-grade solvents with high costs. The expensive equipment and solvents result in prohibitively elevated production costs. Furthermore, the separation procedure is operationally complex and unsuitable for industrial-scale production.

CN114698375A and CN114667284A respectively describe processes of resolving different intermediates to obtain chiral intermediates, followed by further reactions to prepare finerenone via such routes. However, the enantiomeric excess (ee) values of the resolved intermediate diastereomers range from only 65% to 84%. The enantiomers require additional purification, resulting in cumbersome routes and high production costs.

CN115340539A also discloses a preparation method for finerenone. The reaction steps are as follows:

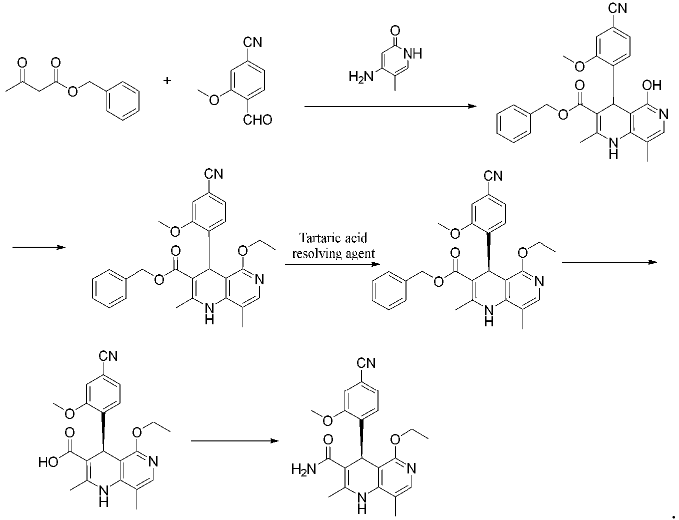

Critical limitations in the routes of the present invention include: (1) In this reaction, difficulty in hydrogenolytic removal of the benzyl group during deprotection, requiring a large amount of palladium on carbon (weight ratio of 10% palladium on carbon to 4-(4-cyano-2-methoxyphenyl)-2,8-dimethyl-5-ethoxy-1,4,5,6-tetrahydro-1,6-naphthyr idine-3-carboxylic acid 2-benzyl ester is 0.1:1). This leads to high reaction costs. (2) Actual repeated implementation of this protocol confirms excessive generation of over-reduced impurities (dihydropyridine ring impurities accounting for over 15%) during hydrogenation, with actual yield below 80%. These impurities are difficult to remove and severely compromise final product quality. (3) Low efficiency of tartaric acid-mediated resolution for the intermediate. Repeated verification demonstrates that a single resolution achieves enantiomeric excess (ee) values of only 75% to 85%. Multiple purification cycles are required to achieve >99% ee values, severely reducing yield and production efficiency.

### SUMMARY

To address the deficiencies in the prior art, the present invention provides a finerenone intermediate compound V, a preparation method thereof, and a preparation method of finerenone.

A finerenone intermediate compound V, wherein the structural formula of the finerenone intermediate compound V is:

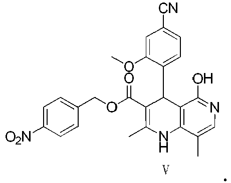

A method for preparing the finerenone intermediate compound V, comprising the following steps: 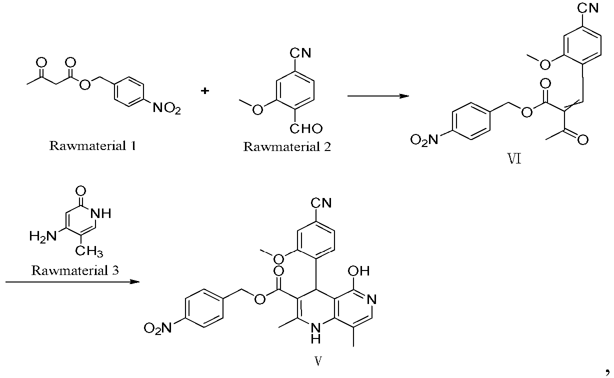 adding 4-cyano-2-methoxybenzaldehyde (raw material 2) and p-nitrobenzyl acetoacetate (raw material 1) to dichloromethane, then adding piperidine and glacial acetic acid, heating to reflux for water separation, cooling the reaction mixture after completion of the reaction, adding purified water, washing and separating layers, drying with anhydrous sodium sulfate, filtering, concentrating the filtrate to dryness to obtain a yellow solid residue (compound VI), adding 2-butanol to the residue, then adding 4-amino-5-methyl-2-hydroxypyridine (raw material 3), heating to reflux, cooling the reaction mixture after completion of the reaction, stirring for crystallization, filtering, washing the filter cake with isopropanol, and subjecting to air-blast drying to obtain compound V.

Additionally, the present invention encompasses a method for preparing finerenone from compound V, primarily implemented through two routes:

Route 1 is as follows: 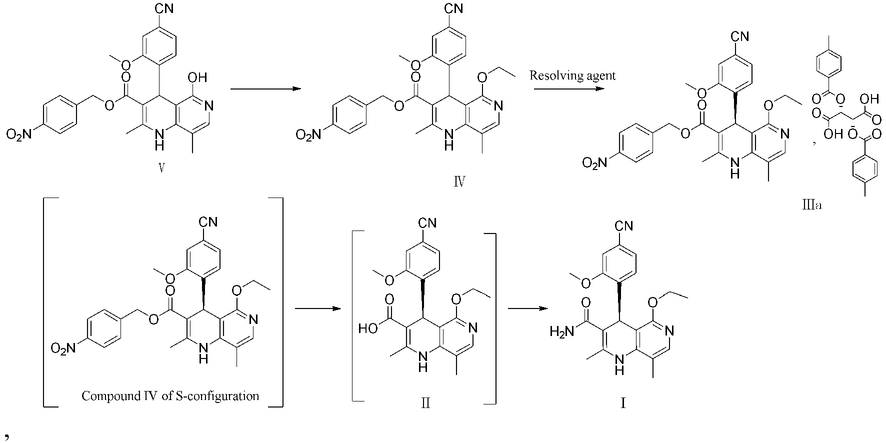
(1) adding compound V and triethyl orthoacetate to N-methylpyrrolidone, followed by dropwise addition of concentrated sulfuric acid; heating the mixture for reaction; after reaction completion, cooling the reaction mixture, adding purified water dropwise, stirring after completion of dropwise addition, and then adding additional purified water; cooling and stirring the mixture, followed by continued cooling and stirring; and filtering and subjecting to air-blast drying to obtain compound IV;
(2) adding compound IV to an organic solvent, then adding L-di-p-toluoyl tartaric acid, heating the mixture, reacting, cooling, maintaining the mixture with stirring at constant temperature, filtering, washing the filter cake with ethyl acetate, and drying to obtain compound IIIa; and
(3) preparing finerenone:
   adding compound IIIa to dichloromethane, adjusting to alkalinity with a 3% sodium bicarbonate aqueous solution, extracting, separating layers, and distilling, adding tetrahydrofuran to the distillation residue, adding 5% palladium on carbon, performing a hydrogenation reaction, filtering to obtain a tetrahydrofuran solution of compound II, further adding 4-dimethylaminopyridine and N,N'-carbonyldiimidazole, reacting, then adding ammonia water, crystallizing, filtering, recrystallizing with anhydrous ethanol, filtering, and drying to obtain finerenone.

In step (2), a molar ratio of compound IV to L-di-p-toluoyl tartaric acid is 1:0.5 to 1.3.

In step (2), the organic solvent is ethyl acetate, methanol, ethanol, isopropanol, dichloromethane, or a combination thereof.

In step (3), a weight ratio of palladium on carbon to compound IV of S-configuration is 0.2% to 2.5%:1.

Route 2 is as follows: 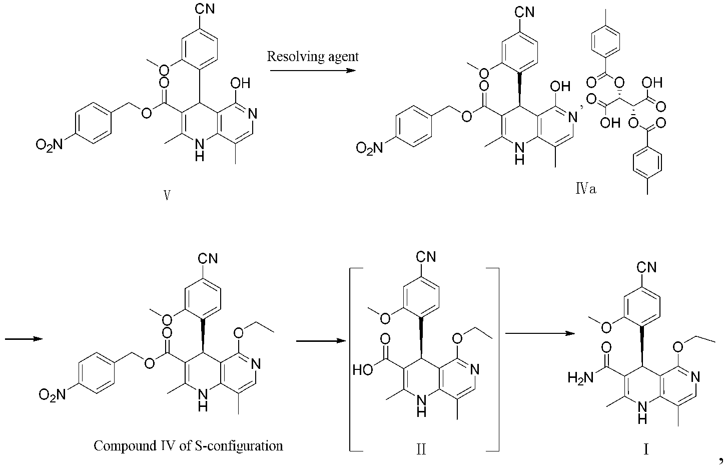
(1) resolving compound V to compound IVa:
   adding compound V to an organic solvent, then adding L-di-p-toluoyl tartaric acid to the organic solution, heating the mixture, reacting, cooling, maintaining the mixture with stirring at constant temperature, filtering, washing the filter cake with ethyl acetate, and drying to obtain resolved salt compound IVa; and
(2) preparing finerenone:
   adding compound IVa to dichloromethane, adjusting to alkalinity with a 3% sodium bicarbonate aqueous solution, extracting, separating layers, and concentrating to obtain a residue, adding N-methylpyrrolidone, triethyl orthoacetate, and concentrated sulfuric acid to the residue, reacting to obtain compound IV of S-configuration, then adding tetrahydrofuran, adding 5% palladium on carbon, performing a hydrogenation reaction, filtering to obtain a tetrahydrofuran solution of compound II, further adding 4-dimethylaminopyridine and N,N'-carbonyldiimidazole, reacting, then adding ammonia water, crystallizing, filtering, recrystallizing with anhydrous ethanol, filtering, and drying to obtain finerenone.

In step (1), a molar ratio of compound V to L-di-p-toluoyl tartaric acid is 1:0.5 to 1.3.

In step (1), the organic solvent is ethyl acetate, methanol, ethanol, isopropanol, dichloromethane, or a combination thereof.

In step (2), a weight ratio of palladium on carbon to compound IV of S-configuration is 0.2% to 2.5%:1.

**Beneficial effects of the present invention:**
(1) The inventors creatively invented a key finerenone intermediate (compound V). Through extensive experimental exploration, it was found that compound V is a superior intermediate with stable and controllable quality, more favorable for controlling finerenone quality and production costs. By introducing a nitro substituent, compound V exhibits increased molecular polarity. This modification facilitates cocrystal formation with L-di-p-toluoyl tartaric acid and is more favorable for resolution. The resolved intermediate achieves >99% ee value with a resolution yield of 45% based on the racemate. The obtained intermediate has high purity and can be directly used in the subsequent reaction. Studies found that when chlorine is substituted on the phenyl ring, the molecular polarity changes little. The resulting cocrystals with L-di-p-toluoyl tartaric acid exhibit relatively high solubility, leading to poor resolution effect of the intermediate (ee value: merely 80%-85%).
(2) In Route 2 of the present invention, the resolution step is performed earlier. Performing resolution prior to reaction with triethyl orthoacetate provides good atom economy.
(3) In preparing the finerenone final product, the present invention employs a one-pot reaction to directly obtain the target product. This approach reduces post-reaction purification requirements, minimizes generation of three wastes, simplifies operational procedures, and is more conducive to scale-up production while lowering manufacturing costs.
(4) The inventors creatively discovered that performing hydrogenation on compound IV of S-configuration utilizes the strong electron-withdrawing nature of the nitro group to render the entire molecular structure electron-deficient, thereby fundamentally preventing over-reduction of the dihydropyridine ring in the product under hydrogenation conditions. Simultaneously, the amount of catalyst (5% palladium on carbon) is maintained at 0.2% to 2.5% of the input amount of compound IV of S-configuration. This optimization enhances product quality while substantially reducing production costs.
(5) The preparation method of finerenone according to the present invention provides finerenone with a purity of 99.9% and an ee value of 99.9%.

### BRIEF DESCRIPTION OF THE DRAWINGS

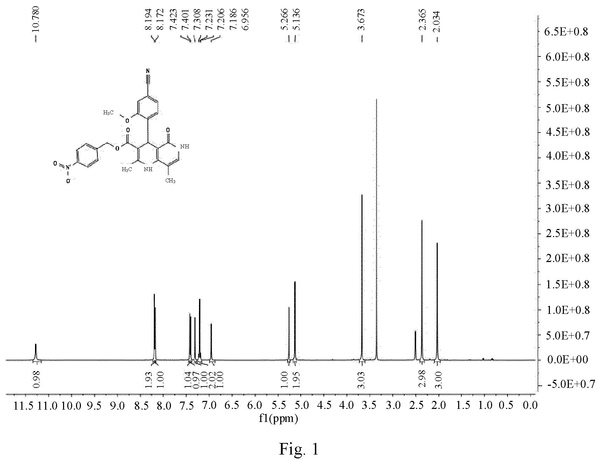

Fig. 1 shows the 1H-NMR spectrum of compound V.

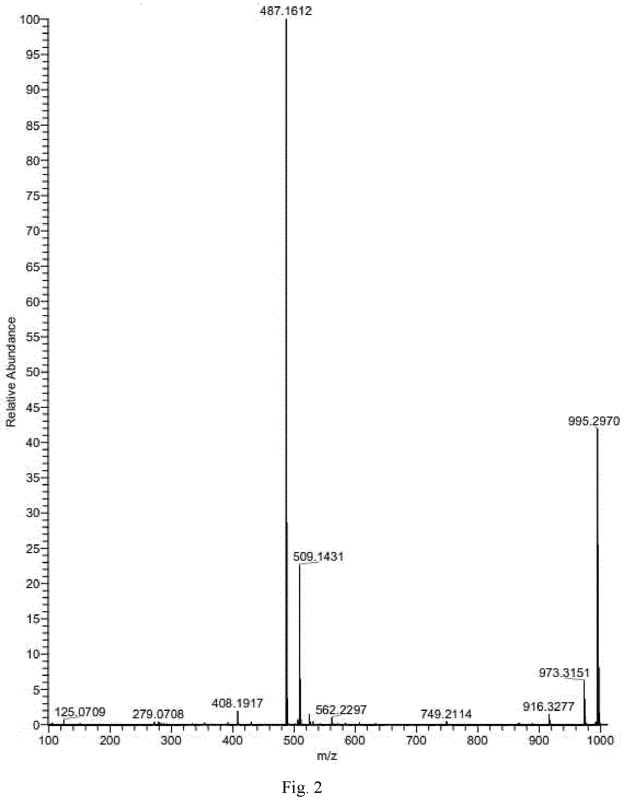

Fig. 2 shows the mass spectrometry (MS) spectrum of compound V.

### DETAILED DESCRIPTION OF THE EMBODIMENTS

The present invention is further described below with reference to the drawings and specific embodiments, so that those skilled in the art may better understand the present invention, while this does not limit the present invention.

### Embodiment 1: Preparation of compound V

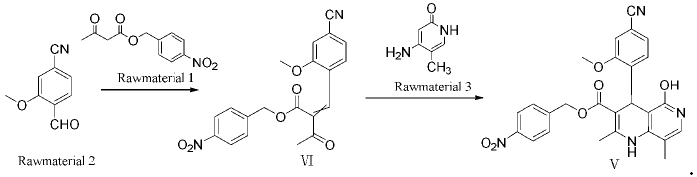

To a reaction flask were added 400 mL of dichloromethane, followed by 4-cyano-2-methoxybenzaldehyde (40 g, 248 mmol) and p-nitrobenzyl acetoacetate (70.6 g, 296 mmol). Piperidine (2.0 g) and glacial acetic acid (1.4 g) were then added. The mixture was heated to reflux for water separation and reacted for 6 hours. After completion of the reaction, the mixture was cooled. Purified water (100 mL) was added and the mixture was washed twice. The mixture was dried over anhydrous sodium sulfate and filtered. The filtrate was concentrated to obtain a yellow solid. 2-Butanol (600 mL) and 4-amino-5-methyl-2-hydroxypyridine (27.8 g, 224 mmol) were added to the yellow solid. The mixture was heated to reflux and reacted for 20 hours, then cooled to 0°C and stirred for crystallization for 2 hours. The yellow solid was filtered and washed with 20 mL of isopropanol. The yellow solid was dried by air-blast drying at 50°C to obtain 90.4 g of compound V, with a purity of 98.5% and a yield of 83.0% (based on 4-amino-5-methyl-2-hydroxypyridine).

1H-NMR (400MHz, DMSO-d6) δ = 2.034 (s, 3H), δ = 2.365 (s, 3H), δ = 3.673 (s, 3H), δ = 5.136 (s, 2H), δ = 5.266 (s, 1H), δ = 6.956 (s, 1H), δ = 7.186-7.231 (m, 2H), δ = 7.308 (s, 1H), δ = 7.401 (s, 1H), δ = 7.423 (s, 1H), δ = 8.172 (s, 1H), δ = 8.194 (s, 2H), δ = 10.780 (s, 1H).

MS (ESI): m/z = 487.2 [M+H]⁺.

### Embodiment 2:

### Route 1: Preparation Method of Finerenone

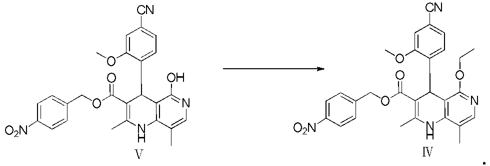

To a reaction flask were added 68 mL of N-methylpyrrolidone, followed by compound V (45 g, 92 mmol) and triethyl orthoacetate (45.0 g, 277 mmol). Concentrated sulfuric acid (2.7 g) was then added dropwise. The mixture was heated to 115°C and reacted for 2 hours, and then cooled to 50°C. Purified water (68 mL) was added dropwise. After the dropwise addition was complete, the mixture was stirred for 0.5 hours, followed by dropwise addition of another 68 mL of purified water. The mixture was further cooled to 0°C and stirred for crystallization for 1 hour. The mixture was filtered and the filter cake was dried by air-blast drying at 50°C to obtain 43.2 g of compound IV, with a yield of 90.8% and a purity of 98.8%.

1H-NMR (400MHz, DMSO-d6) δ = 1.162-1.197 (t, 3H), δ = 1.602 (s, 3H), δ = 2.151 (s, 3H), δ = 3.640 (s, 3H), δ = 4.095-4.183 (m, 2H), δ = 5.132 (s, 2H), δ = 5.525 (s, 1H), δ = 5.992 (s, 1H), δ = 6.972 (s, 1H), δ = 7.086-7.108 (dd, 1H), δ = 7.263 (s, 1H), δ = 7.291-7.320 (m, 3H), δ = 8.154 (s, 1H), δ = 8.175 (s, 1H).

MS (ESI): m/z = 515.2 [M+H]⁺.

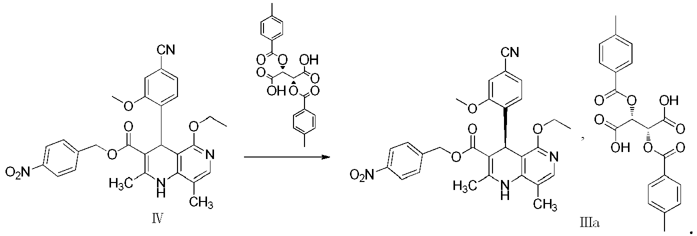

To a reaction flask were added 800 mL of ethyl acetate, followed by compound IV (40 g, 78 mmol) and L-di-p-toluoyl tartaric acid (15 g, 39 mmol). The mixture was heated to 70°C and stirred at this temperature for 1 hour to dissolve. The mixture was then cooled to 30°C and stirred for 2 hours. The mixture was filtered, and the filter cake was washed with 40 mL of ethyl acetate. The filter cake was dried by air-blast drying at 50°C to obtain 32.7 g of compound IIIa, with a resolution yield of 46.7% and an ee value of 99.9%.

1H-NMR (400MHz, DMSO-d6) δ = 1.162-1.197 (t, 3H), δ = 1.602 (s, 3H), δ = 2.151 (s, 3H), δ = 2.409 (s, 6H), δ = 3.640 (s, 3H), δ = 4.095-4.183 (m, 2H), δ = 5.132 (s, 2H), δ = 5.525 (s, 1H), δ = 5.829 (s, 2H), δ = 5.992 (s, 1H), δ = 6.972 (s, 1H), δ = 7.086-7.108 (dd, 1H), δ = 7.263 (s, 1H), δ = 7.291-7.320 (m, 3H), δ = 7.396 (s, 2H), δ = 7.417 (s, 2H), δ = 7.895 (s, 2H), δ = 7.915 (s, 2H), δ = 8.154 (s, 1H), δ = 8.175 (s, 1H), δ = 13.885 (s, 2H).

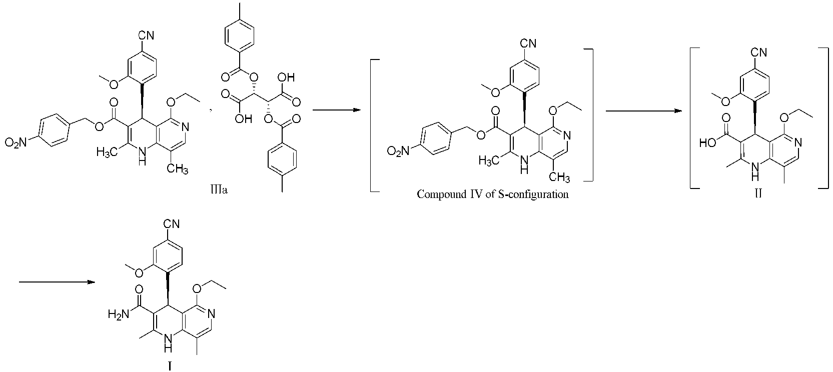

To a reaction flask were added 250 mL of dichloromethane, followed by compound IIIa (25 g, 27.7 mmol). A 3% aqueous sodium carbonate solution was added to adjust the pH to 7-8. After the pH adjustment, the solution became clear, and the organic layer was separated and washed with 100 mL of purified water. The organic layer was dried over anhydrous sodium sulfate and concentrated, and after concentration was completed, a residue was obtained. Tetrahydrofuran (150 mL) was added to the residue and the mixture was stirred to dissolve. Then, 5% Palladium on carbon (0.36 g) was added. The system was purged with hydrogen three times, and the mixture was stirred at 10°C for 1 hour. The mixture was filtered. 4-Dimethylaminopyridine (0.32 g, 2.6 mmol) was added, followed by the addition of N,N'-carbonyldiimidazole (6.0 g, 37 mmol) with stirring. The mixture was stirred at room temperature for 3 hours. Aqueous ammonia solution (30 mL) was then slowly added, and the mixture was stirred at 50°C for 8 hours. After completion of the reaction, the mixture was cooled to 20°C. Purified water (150 mL) was added dropwise, and the mixture was slowly cooled to 0°C and stirred for crystallization for 1 hour. The mixture was filtered. The filter cake was added to 150 mL of anhydrous ethanol, heated to 75°C, and stirred for 1 hour. The mixture was then slowly cooled to 20°C and stirred for crystallization for 1 hour. The mixture was filtered and the filter cake was dried to obtain 9.7 g of finerenone, with a yield of 92.4%, a purity of 99.9%, and an ee value of 99.9%.

1H-NMR (400MHz, DMSO-d6): δ = 1.032-1.067 (t, 3H), δ = 2.122 (s, 3H), δ = 2.188 (s, 3H), δ = 3.825 (s, 3H), δ = 3.958-4.035 (m, 2H), δ = ,6.661-6.749 (d, 2H), δ = 2.122 (s, 3H), δ = 7.142-7.161 (d, 1H), δ = 7.2,1-7.290 (d, 1H), δ = 7.371 (s, 1H), δ = 7.554 (s, 1H), δ = 7.685 (s, 1H).

MS (ESI): m/z = 379.1 [M+H]⁺.

### Embodiment 3:

### Route 2: Preparation Method of Finerenone

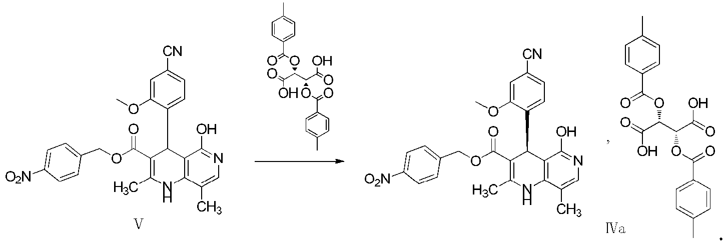

To a reaction flask were added 800 mL of dichloromethane, followed by compound V (40 g, 82 mmol) and L-di-p-toluoyl tartaric acid (41.3 g, 107 mmol). The mixture was heated to 35°C and stirred at this temperature for 1 hour to dissolve. The mixture was then cooled to 20°C and stirred for 2 hours. The mixture was filtered, and the filter cake was washed with 40 mL of ethyl acetate. The filter cake was dried by air-blast drying at 50°C to obtain 31.5 g of compound IVa, with a resolution yield of 43.9% and an ee value of 99.9%.

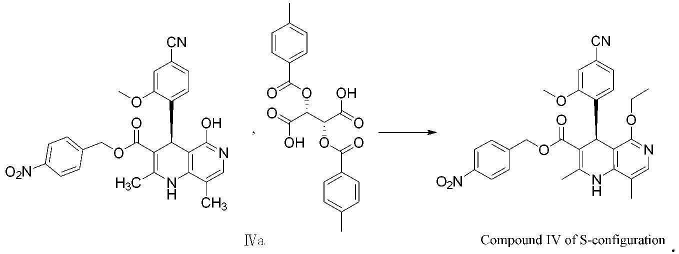

To a reaction flask were added 250 mL of dichloromethane, followed by compound IVa (30 g, 34 mmol). A 3% aqueous sodium carbonate solution was added to adjust the pH to 7-8. After the pH adjustment, the solution became clear, and the organic layer was separated and washed with 100 mL of purified water. The organic layer was dried over anhydrous sodium sulfate and concentrated, and after concentration was completed, a residue was obtained. N-Methylpyrrolidone (75 mL) was added to the residue, followed by triethyl orthoacetate (16.5 g, 102 mmol). Concentrated sulfuric acid (1 g) was then added dropwise. The mixture was heated to 115°C and reacted for 2 hours, then cooled to 50°C. Purified water (25 mL) was added dropwise. After the dropwise addition was complete, the mixture was stirred for 0.5 hours, followed by dropwise addition of another 75 mL of purified water. The mixture was further cooled to 0°C and stirred for crystallization for 1 hour. The mixture was filtered and the filter cake was dried by air-blast drying at 50°C to obtain 15.8 g of compound IV of S-configuration, with a yield of 89.3% and a purity of 98.8%.

1H-NMR (400MHz, DMSO-d6) δ = 1.162-1.197 (t, 3H), δ = 1.602 (s, 3H), δ = 2.151 (s, 3H), δ = 3.640 (s, 3H), δ = 4.095-4.183 (m, 2H), δ = 5.132 (s, 2H), δ = 5.525 (s, 1H), δ = 5.992 (s, 1H), δ = 6.972 (s, 1H), δ = 7.086-7.108 (dd, 1H), δ = 7.263 (s, 1H), δ = 7.291-7.320 (m, 3H), δ = 8.154 (s, 1H), δ = 8.175 (s, 1H).

MS (ESI): m/z = 515.2 [M+H]⁺.

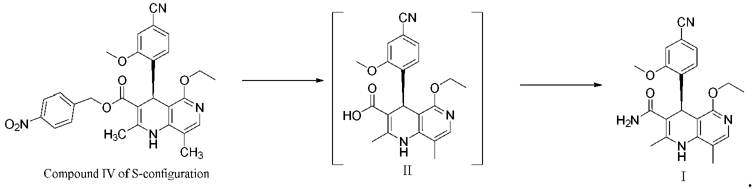

To a reaction flask were added 150 mL of tetrahydrofuran, followed by the S-configuration compound (15 g, 29.1 mmol). The mixture was stirred to dissolve. 5% Palladium on carbon (30 mg) was added. The system was purged with hydrogen three times, and the mixture was stirred at 10°C for 1 hour. The mixture was filtered. 4-Dimethylaminopyridine (0.32 g, 2.6 mmol) was added, followed by the addition of N,N'-carbonyldiimidazole (6 g, 37.0 mmol) with stirring. The mixture was stirred at room temperature for 3 hours. Aqueous ammonia solution (30 mL) was then slowly added, and the mixture was stirred at 50°C for 8 hours. After completion of the reaction, the mixture was cooled to 20°C. Purified water (150 mL) was added dropwise, and the mixture was slowly cooled to 0°C and stirred for crystallization for 1 hour. The mixture was filtered. The filter cake was added to 150 mL of anhydrous ethanol, heated to 75°C, and stirred for 1 hour. The mixture was then slowly cooled to 20°C and stirred for crystallization for 1 hour. The mixture was filtered and the filter cake was dried to obtain 10.1 g of finerenone, with a yield of 91.6%, a purity of 99.8%, and an ee value of 99.9%.

1H-NMR (400MHz, DMSO-d6): δ = 1.032-1.067 (t, 3H), δ = 2.122 (s, 3H), δ = 2.188 (s, 3H), δ = 3.825 (s, 3H), δ = 3.958-4.035 (m, 2H), δ = ,6.661-6.749 (d, 2H), δ = 2.122 (s, 3H), δ = 7.142-7.161 (d, 1H), δ = 7.2,1-7.290 (d, 1H), δ = 7.371 (s, 1H), δ = 7.554 (s, 1H), δ = 7.685 (s, 1H).

MS (ESI): m/z = 379.1 [M+H]⁺.

### Embodiment 4: Preparation of compound V

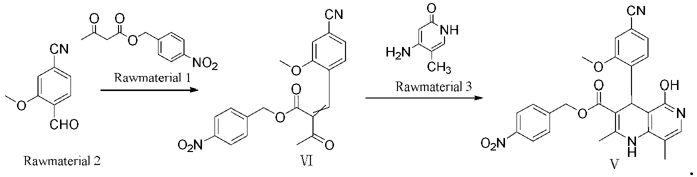

To a reaction vessel were added 40 L of dichloromethane, followed by 4-cyano-2-methoxybenzaldehyde (4 kg, 24.8 mol) and p-nitrobenzyl acetoacetate (7.1 kg, 29.9 mol). Piperidine (200 g) and glacial acetic acid (140 g) were then added. The mixture was heated to reflux for water separation and reacted for 6 hours. After completion of the reaction, the mixture was cooled. Purified water (10 L) was added and the mixture was washed twice. The mixture was dried over anhydrous sodium sulfate and filtered. The filtrate was concentrated to obtain a yellow solid. 2-Butanol (60 L) and 4-amino-5-methyl-2-hydroxypyridine (2.8 kg, 22.6 mol) were added to the yellow solid. The mixture was heated to reflux and reacted for 20 hours, then cooled to 0°C and stirred for crystallization for 2 hours. The yellow solid was filtered and washed with 2 L of isopropanol. The yellow solid was dried by air-blast drying at 50°C to obtain 9.2 kg of compound V, with a purity of 98.7% and a yield of 83.8% (based on 4-amino-5-methyl-2-hydroxypyridine).

MS (ESI): m/z = 487.2 [M+H]⁺.

### Embodiment 5:

### Route 1: Preparation Method of Finerenone

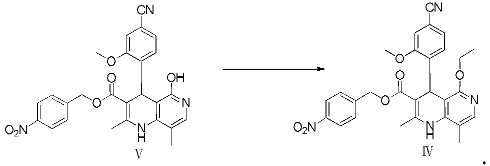

To a reaction vessel were added 6.8 L of N-methylpyrrolidone, followed by compound V (4.5 kg, 9.3 mol) and triethyl orthoacetate (4.5 kg, 27.7 mol). Concentrated sulfuric acid (270 g) was then added dropwise. The mixture was heated to 115°C and reacted for 2 hours, and then cooled to 50°C. Purified water (6.8 L) was added dropwise. After the dropwise addition was complete, the mixture was stirred for 0.5 hours, followed by dropwise addition of another 6.8 L of purified water. The mixture was further cooled to 0°C and stirred for crystallization for 1 hour. The mixture was filtered and the filter cake was dried by air-blast drying at 50°C to obtain 4.41 kg of compound IV, with a yield of 92.7% and a purity of 98.9%.

MS (ESI): m/z = 515.2 [M+H]⁺.

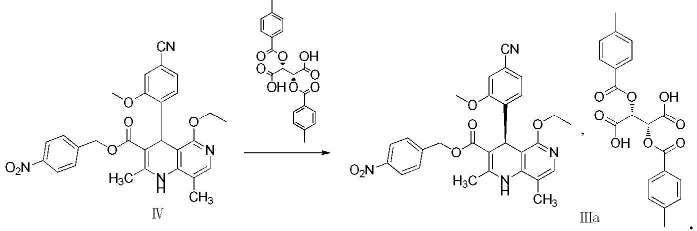

To a reaction vessel were added 80 L of dichloromethane, followed by compound IV (4 kg, 7.8 mol) and L-di-p-toluoyl tartaric acid (3.9 kg, 10.1 mol). The mixture was heated to 35°C and stirred at this temperature for 1 hour to dissolve. The mixture was then slowly cooled to 20°C and stirred for 2 hours. The mixture was filtered, and the filter cake was washed with 4 L of ethyl acetate. The filter cake was dried by air-blast drying at 50°C to obtain 3.3 kg of compound IIIa, with a resolution yield of 47.1% and an ee value of 99.9%.

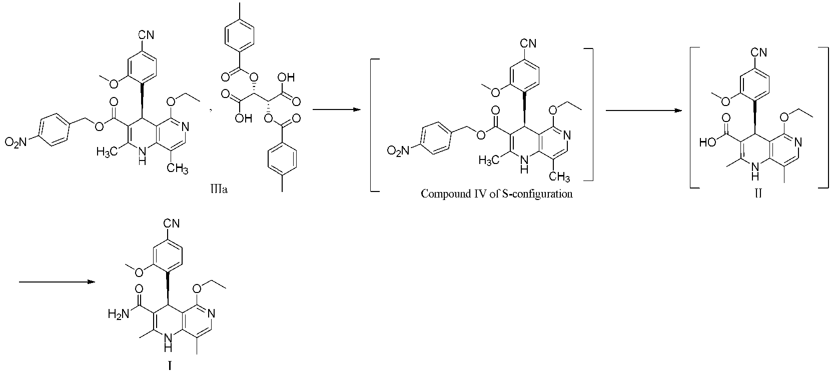

To a reaction vessel were added 25 L of dichloromethane, followed by compound IIIa (2.5 kg, 2.8 mol). A 3% aqueous sodium carbonate solution was added to adjust the pH to 7-8. After the pH adjustment, the solution became clear, and the organic layer was separated and washed with 10 L of purified water. The organic layer was dried over anhydrous sodium sulfate and concentrated, and after concentration was completed, a residue was obtained. Tetrahydrofuran (15 L) was added to the residue and the mixture was stirred to dissolve. Then, 5% Palladium on carbon (2.9 g) was added. The system was purged with hydrogen three times, and the mixture was stirred at 10°C for 1 hour. The mixture was filtered. 4-Dimethylaminopyridine (32 g, 0.26 mol) was added, followed by the addition of N,N'-carbonyldiimidazole (600 g, 3.7 mol) with stirring. The mixture was stirred at room temperature for 3 hours. Aqueous ammonia solution (3 L) was then slowly added, and the mixture was stirred at 50°C for 8 hours. After completion of the reaction, the mixture was cooled to 20°C. Purified water (15 L) was added dropwise, and the mixture was slowly cooled to 0°C and stirred for crystallization for 1 hour. The mixture was filtered. The filter cake was added to 15 L of anhydrous ethanol, heated to 75°C, and stirred for 1 hour. The mixture was then slowly cooled to 20°C and stirred for crystallization for 1 hour. The mixture was filtered and the filter cake was dried to obtain 0.96 kg of finerenone, with a yield of 91.4%, a purity of 99.9%, and an ee value of 99.9%.

MS (ESI): m/z = 379.1 [M+H]⁺.

### Embodiment 6:

### Route 2: Preparation Method of Finerenone

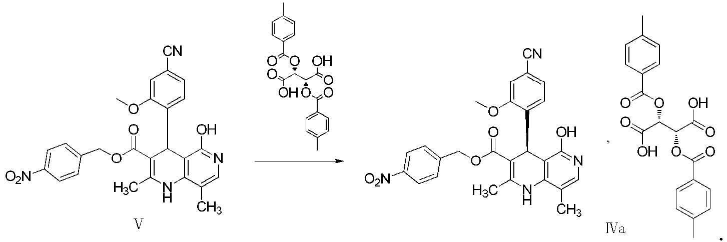

To a reaction vessel were added 80 L of ethanol, followed by compound V (4 kg, 8.2 mol) and L-di-p-toluoyl tartaric acid (1.6 kg, 4.1 mol). The mixture was heated to 75°C and stirred at this temperature for 1 hour to dissolve. The mixture was then cooled to 30°C and stirred for 2 hours. The mixture was filtered, and the filter cake was washed with 4 L of ethyl acetate. The filter cake was dried by air-blast drying at 50°C to obtain 3.1 kg of compound IVa, with a resolution yield of 43.2% and an ee value of 99.9%.

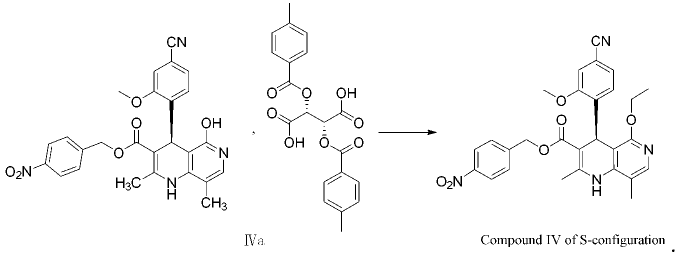

To a reaction vessel were added 25 L of dichloromethane, followed by compound IVa (3 kg, 3.4 mol). A 3% aqueous sodium carbonate solution was added to adjust the pH to 7-8. After the pH adjustment, the solution became clear, and the organic layer was separated and washed with 10 L of purified water. The organic layer was dried over anhydrous sodium sulfate and concentrated, and after concentration was completed, a residue was obtained. N-Methylpyrrolidone (7.5 L) was added to the residue, followed by triethyl orthoacetate (1.6 kg, 9.9 mol). Concentrated sulfuric acid (100 g) was then added dropwise. The mixture was heated to 115°C and reacted for 2 hours, then cooled to 50°C. Purified water (2.5 L) was added dropwise. After the dropwise addition was complete, the mixture was stirred for 0.5 hours, followed by dropwise addition of another 7.5 L of purified water. The mixture was further cooled to 0°C and stirred for crystallization for 1 hour. The mixture was filtered and the filter cake was dried by air-blast drying at 50°C to obtain 1.6 kg of compound IV of S-configuration, with a yield of 90.5% and a purity of 99.0%.

MS (ESI): m/z = 515.2 [M+H]⁺.

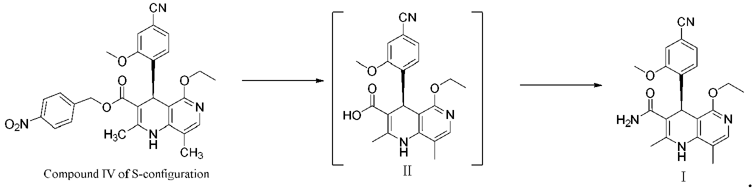

To a reaction vessel were added 15 L of tetrahydrofuran, followed by the S-configuration compound (1.5 kg, 2.9 mol). The mixture was stirred to dissolve. 5% Palladium on carbon (37.5 g) was added. The system was purged with hydrogen three times, and the mixture was stirred at 10°C for 1 hour. The mixture was filtered. 4-Dimethylaminopyridine (32 g, 0.26 mol) was added, followed by the addition of N,N'-carbonyldiimidazole (600 g, 3.7 mol) with stirring. The mixture was stirred at room temperature for 3 hours. Aqueous ammonia solution (3 L) was then slowly added, and the mixture was stirred at 50°C for 8 hours. After completion of the reaction, the mixture was cooled to 20°C. Purified water (15 L) was added dropwise, and the mixture was slowly cooled to 0°C and stirred for crystallization for 1 hour. The mixture was filtered. The filter cake was added to 15 L of anhydrous ethanol, heated to 75°C, and stirred for 1 hour. The mixture was then slowly cooled to 20°C and stirred for crystallization for 1 hour. The mixture was filtered and the filter cake was dried to obtain 1.0 kg of finerenone, with a yield of 90.6%, a purity of 99.9%, and an ee value of 99.9%.

MS (ESI): m/z = 379.1 [M+H]⁺.

### Comparative Embodiment 1

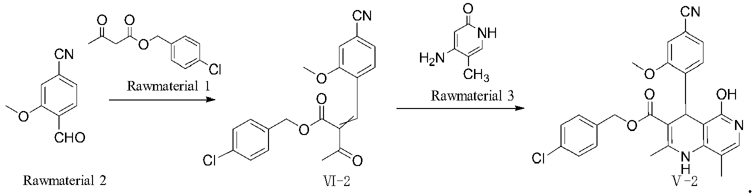

To a reaction flask were added 40 mL of dichloromethane, followed by 4-cyano-2-methoxybenzaldehyde (4.0 g, 24 mmol) and p-chlorobenzyl acetoacetate (6.8 g, 30 mmol). Piperidine (0.2 g) and glacial acetic acid (0.14 g) were then added. The mixture was heated to reflux for water separation and reacted for 6 hours. After completion of the reaction, the mixture was cooled. Purified water (10 mL) was added and the mixture was washed twice. The mixture was dried over anhydrous sodium sulfate and filtered. The filtrate was concentrated to obtain a yellow solid. 2-Butanol (60 mL) and 4-amino-5-methyl-2-hydroxypyridine (2.8 g, 22 mmol) were added to the yellow solid. The mixture was heated to reflux and reacted for 20 hours, then cooled to 0°C and stirred for crystallization for 2 hours. The yellow solid was filtered and washed with 10 mL of isopropanol. The yellow solid was dried by air-blast drying at 50°C to obtain 8.2 g of compound V-2, with a purity of 98.2% and a yield of 76.4% (based on 4-amino-5-methyl-2-hydroxypyridine).

1H-NMR (400MHz, DMSO-d6) δ = 2.032 (s, 3H), δ = 2.361 (s, 3H), δ = 3.672 (s, 3H), δ = 5.135 (s, 2H), δ = 5.265 (s, 1H), δ = 6.904 (s, 1H), δ = 7.086-7.130 (m, 2H), δ = 7.201 (s, 1H), δ = 7.305 (s, 1H), δ = 7.420 (s, 1H), δ = 8.160 (s, 1H), δ = 8.181 (s, 2H), δ = 10.782 (s, 1H).

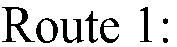

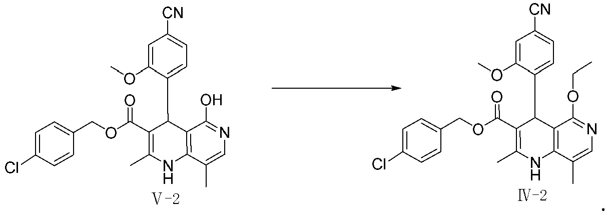

To a reaction flask were added 6 mL of N-methylpyrrolidone, followed by compound V-2 (4 g, 8.4 mmol) and triethyl orthoacetate (4.1 g, 25 mmol). Concentrated sulfuric acid (0.2 g) was then added dropwise. The mixture was heated to 115°C and reacted for 2 hours, then cooled to 50°C. Purified water (6 mL) was added dropwise. After the dropwise addition was complete, the mixture was stirred for 0.5 hours, followed by dropwise addition of another 6 mL of purified water. The mixture was further cooled to 0°C and stirred for crystallization for 1 hour. The mixture was filtered and the filter cake was dried by air-blast drying at 50°C to obtain 3.8 g of compound IV-2, with a yield of 89.8% and a purity of 98.5%.

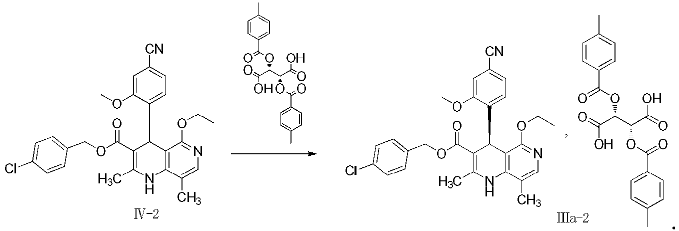

To a reaction flask were added 40 mL of ethyl acetate, followed by compound IV-2 (2 g, 3.9 mmol) and L-di-p-toluoyl tartaric acid (1.5 g, 3.9 mmol). The mixture was heated to 70°C and stirred at this temperature for 1 hour to dissolve. The mixture was then allowed to cool naturally to 25°C and stirred for 2 hours. The mixture was filtered, and the filter cake was washed with 2 mL of ethanol. The filter cake was dried by air-blast drying at 50°C to obtain 1.28 g of compound IIIa-2, with a resolution yield of 36.2% and an ee value of 83.1%.

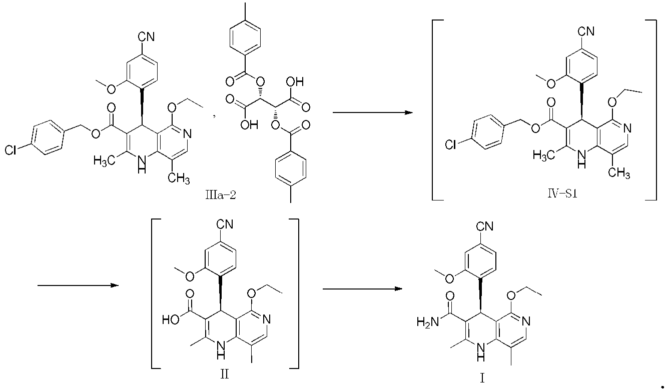

To a reaction flask were added 15 mL of dichloromethane, followed by compound IIIa-2 (1.5 g, 1.7 mmol). A 3% aqueous sodium carbonate solution was added to adjust the pH to 7-8. After the pH adjustment, the solution became clear, and the organic layer was separated and washed with 7 mL of purified water. The organic layer was dried over anhydrous sodium sulfate and concentrated, and after concentration was completed, a residue was obtained. Tetrahydrofuran (10 mL) was added to the residue. Then, 10% Palladium on carbon (25 mg) was added. The system was purged with hydrogen three times, and the mixture was stirred at 10°C for 1 hour. The mixture was filtered. 4-Dimethylaminopyridine (21 mg, 0.2 mmol) was added, followed by the addition of N,N'-carbonyldiimidazole (0.4 g, 2.5 mmol) with stirring. The mixture was stirred at room temperature for 3 hours. Aqueous ammonia solution (2 mL) was then slowly added, and the mixture was stirred at 50°C for 8 hours. After completion of the reaction, the mixture was cooled to 20°C. Purified water (10 mL) was added dropwise, and the mixture was slowly cooled to 0°C and stirred for crystallization for 1 hour. The mixture was filtered. The filter cake was added to 5 mL of anhydrous ethanol, heated to 75°C, and stirred for 1 hour. The mixture was then slowly cooled to 20°C and stirred for crystallization for 1 hour. The mixture was filtered and the filter cake was dried to obtain 0.59 g of finerenone, with a yield of 94.6%, a purity of 92.5%, and an ee value of 83.5%.

MS (ESI): m/z = 379.1 [M+H]⁺.

### Comparative Embodiment 2

### Method 2

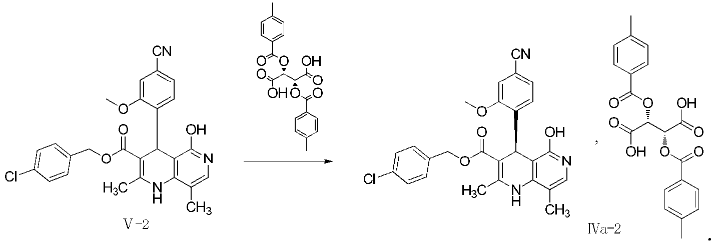

To a reaction flask were added 80 mL of dichloromethane, followed by compound V-2 (4 g, 8.4 mmol) and L-di-p-toluoyl tartaric acid (4.2 g, 10.9 mmol). The mixture was heated to 75°C and stirred at this temperature for 1 hour to dissolve. The mixture was then allowed to cool naturally to 25°C and stirred for 2 hours. The mixture was filtered, and the filter cake was washed with 4 mL of ethyl acetate. The filter cake was dried by air-blast drying at 50°C to obtain 2.8 g of compound IVa-2, with a resolution yield of 38.6% and an ee value of 80.8%.

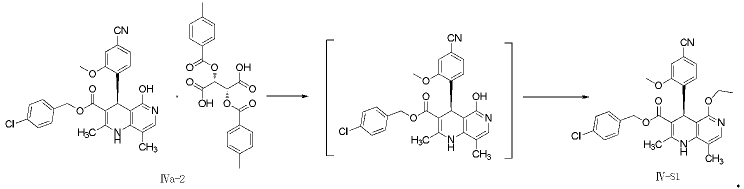

To a reaction flask were added 20 mL of dichloromethane, followed by compound IVa-2 (2 g, 2.3 mmol). A 3% aqueous sodium carbonate solution was added to adjust the pH to 7-8. After the pH adjustment, the solution became clear, and the organic layer was separated and washed with 10 mL of purified water. The organic layer was dried over anhydrous sodium sulfate and concentrated, and after concentration was completed, a residue was obtained. N-Methylpyrrolidone (6 mL) was added to the residue, followed by triethyl orthoacetate (1.1 g, 6.9 mmol). Concentrated sulfuric acid (0.1 g) was then added dropwise. The mixture was heated to 115°C and reacted for 2 hours, and then cooled to 50°C. Purified water (3 mL) was added dropwise. After the dropwise addition was complete, the mixture was stirred for 0.5 hours, followed by dropwise addition of another 3 mL of purified water. The mixture was further cooled to 0°C and stirred for crystallization for 1 hour. The mixture was filtered and the filter cake was dried by air-blast drying at 50°C to obtain 1.01 g of compound IV-S1, with a yield of 86.4% and a purity of 98.8%.

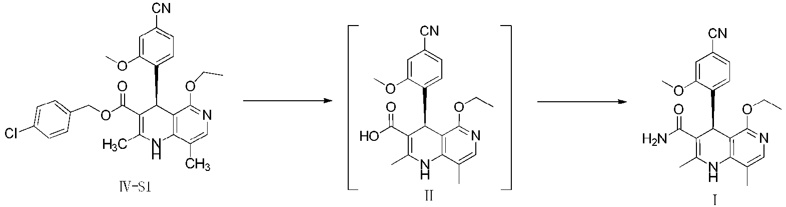

To a reaction flask were added 10 mL of tetrahydrofuran. compound IV-S (1.0 g) was added to the reaction flask and the mixture was stirred to dissolve. Then, 5% Palladium on carbon (25 mg) was added. The system was purged with hydrogen three times, and the mixture was stirred at 10°C for 1 hour. The mixture was filtered. 4-Dimethylaminopyridine (21 mg, 0.2 mmol) was added, followed by the addition of N,N'-carbonyldiimidazole (0.4 g, 2.5 mmol) with stirring. The mixture was stirred at room temperature for 3 hours. Aqueous ammonia solution (2 mL) was then slowly added, and the mixture was stirred at 50°C for 8 hours. After completion of the reaction, the mixture was cooled to 20°C. Purified water (10 mL) was added dropwise, and the mixture was slowly cooled to 0°C and stirred for crystallization for 1 hour. The mixture was filtered. The filter cake was added to 5 mL of anhydrous ethanol, heated to 75°C, and stirred for 1 hour. The mixture was then slowly cooled to 20°C and stirred for crystallization for 1 hour. The mixture was filtered and the filter cake was dried to obtain 0.71 g of finerenone, with a yield of 94.6%, a purity of 88.5%, and an ee value of 81.0%.

1H-NMR (400MHz, DMSO-d6): δ = 1.032-1.067 (t, 3H), δ = 2.122 (s, 3H), δ = 2.188 (s, 3H), δ = 3.825 (s, 3H), δ = 3.958-4.035 (m, 2H), δ = ,6.661-6.749 (d, 2H), δ = 2.122 (s, 3H), δ = 7.142-7.161 (d, 1H), δ = 7.2,1-7.290 (d, 1H), δ = 7.371 (s, 1H), δ = 7.554 (s, 1H), δ = 7.685 (s, 1H).

MS (ESI): m/z = 379.1 [M+H]⁺.

### Comparative Embodiment 3

### Preparation of compound II with reference to Patent CN115340539:

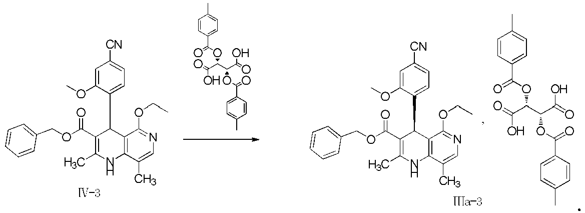

To a reaction flask were added compound IV-3 (10 g, 21.2 mmol), L-di-p-toluoyl tartaric acid (8.2 g, 21.2 mmol), and methyl isobutyl ketone (150 mL) at room temperature (approximately 20°C). The mixture was heated to an internal temperature of 70°C until a clear solution was obtained and stirred for 3 hours. Subsequently, the mixture was cooled to 20°C over 5 hours and stirred at this temperature for 15 hours. The mixture was filtered, and the solid was rinsed with methyl isobutyl ketone (5 mL). The solid was vacuum-dried at 45°C to obtain 9.8 g of white solid compound IIIa-3, with an ee value of 83.1%.

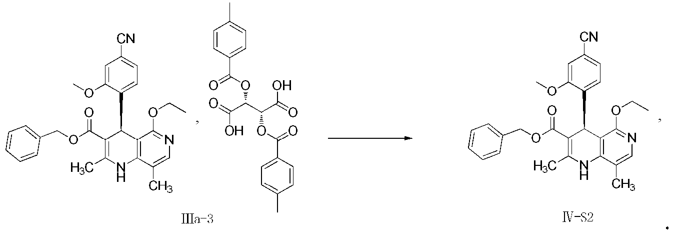

To a reaction flask were added compound IIIa-3 (5.0 g) and water (50 mL) at room temperature (approximately 20°C) and stirred for 30 minutes. An aqueous sodium phosphate solution (100 g of sodium phosphate dissolved in 1000 mL of water) was added dropwise to adjust the pH to 7-7.5. Stirring was continued for 3 hours. The mixture was filtered, and the solid was rinsed with water (20 mL). The solid was vacuum-dried at 45°C to obtain 2.5 g of white solid compound IV-S2, with a yield of 93.6%, a purity of 97.9%, and an ee value of 83.1%.

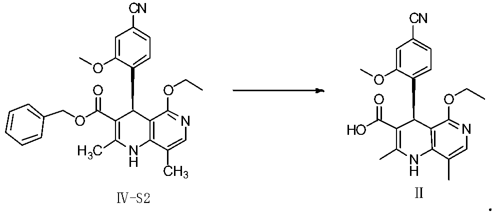

To a reaction flask were added compound IV-S2 (2.5 g, 5.3 mmol), tetrahydrofuran (25 mL), and 10% palladium on carbon (0.25 g) at room temperature (approximately 20°C). The system was purged with nitrogen three times and then with hydrogen three times. Stirring was continued at room temperature for 3 hours. The mixture was filtered to remove the palladium on carbon, and the organic phase was concentrated to obtain 1.85 g of white solid compound II, with a purity of 75.79%.

Experimental repetition under the conditions described in this literature failed to achieve the reported purity of 99.7%. It was found that an impurity (16.8%) was generated and identified as resulting from reduction of the dihydropyridine ring. This impurity affects product quality.

## Claims

1. A finerenone intermediate compound V, **characterized in that** the structural formula of the finerenone intermediate compound V is:

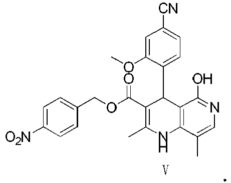

2. A method for preparing the finerenone intermediate compound V according to claim 1, **characterized by** comprising the following steps: 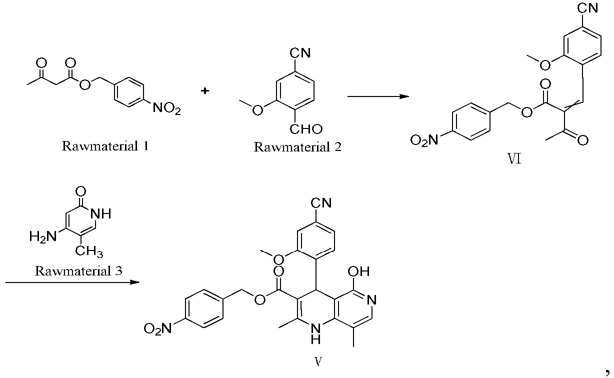 adding 4-cyano-2-methoxybenzaldehyde (raw material 2) and p-nitrobenzyl acetoacetate (raw material 1) to dichloromethane, then adding piperidine and glacial acetic acid, heating to reflux for water separation, cooling the reaction mixture after completion of the reaction, adding purified water, washing and separating layers, drying with anhydrous sodium sulfate, filtering, concentrating the filtrate to dryness to obtain a yellow solid residue (compound VI), adding 2-butanol to the residue, then adding 4-amino-5-methyl-2-hydroxypyridine (raw material 3), heating to reflux, cooling the reaction mixture after completion of the reaction, stirring for crystallization, filtering, washing the filter cake with isopropanol, and subjecting to air-blast drying to obtain compound V.

3. A method for preparing finerenone, **characterized by** comprising the following steps: 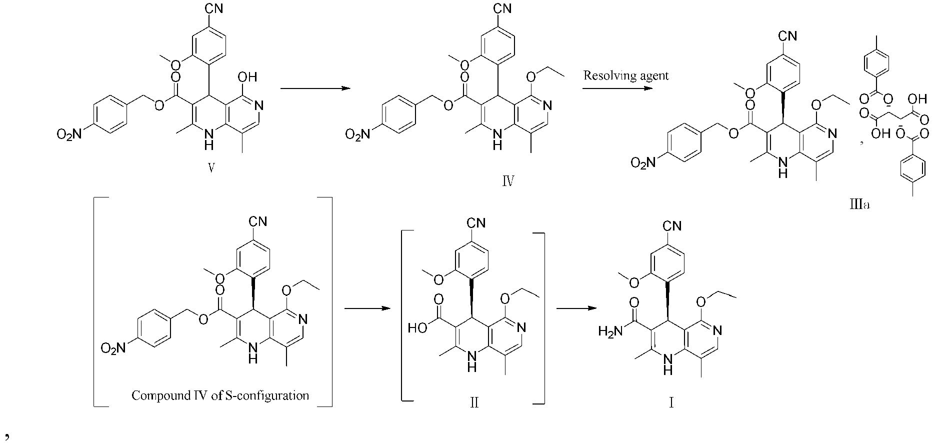
(1) adding compound V, triethyl orthoacetate, and concentrated sulfuric acid to N-methylpyrrolidone, reacting to prepare compound IV;
(2) adding compound IV to an organic solvent, adding L-di-p-toluoyl tartaric acid to the resulting solution, and performing resolution to obtain compound IIIa; and
(3) preparing finerenone:
adding compound IIIa to dichloromethane, adjusting to alkalinity with a sodium bicarbonate aqueous solution, extracting, separating layers, and distilling, adding tetrahydrofuran to the distillation residue, adding 5% palladium on carbon, performing a hydrogenation reaction, filtering to obtain a tetrahydrofuran solution of compound II, further adding 4-dimethylaminopyridine and N,N'-carbonyldiimidazole, reacting, then adding ammonia water, crystallizing, filtering, recrystallizing with anhydrous ethanol, filtering, and drying to obtain finerenone.

4. The method for preparing finerenone according to claim 3, **characterized in that** in step (2), a molar ratio of compound IV to L-di-p-toluoyl tartaric acid is 1:0.5 to 1.3.

5. The method for preparing finerenone according to claim 3, **characterized in that** in step (2), the organic solvent is ethyl acetate, methanol, ethanol, isopropanol, dichloromethane, or a combination thereof.

6. The method for preparing finerenone according to claim 3, **characterized in that** in step (3), a weight ratio of palladium on carbon to compound IV of S-configuration is 0.2% to 2.5%:1.

7. A method for preparing finerenone, **characterized by** comprising the following steps: 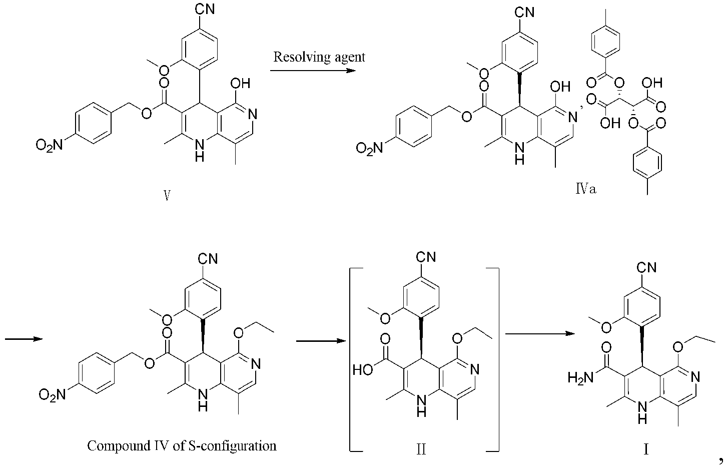
(1) resolving compound V to compound IVa:
adding compound V to a solvent, adding L-di-p-toluoyl tartaric acid to the organic solution, and performing resolution to obtain compound IVa; and
(2) preparing finerenone:
adding compound IVa to dichloromethane, adjusting to alkalinity with a sodium bicarbonate aqueous solution, extracting, separating layers, and concentrating to obtain a residue, adding N-methylpyrrolidone, triethyl orthoacetate, and concentrated sulfuric acid to the residue, reacting to obtain compound IV of S-configuration, then adding tetrahydrofuran, adding 5% palladium on carbon, performing a hydrogenation reaction, filtering to obtain a tetrahydrofuran solution of compound II, further adding 4-dimethylaminopyridine and N,N'-carbonyldiimidazole, reacting, then adding ammonia water, crystallizing, filtering, recrystallizing with anhydrous ethanol, filtering, and drying to obtain finerenone.

8. The method for preparing finerenone according to claim 7, **characterized in that** in step (1), a molar ratio of compound V to L-di-p-toluoyl tartaric acid is 1:0.5 to 1.3.

9. The method for preparing finerenone according to claim 7, **characterized in that** in step (1), the organic solvent is ethyl acetate, methanol, ethanol, isopropanol, dichloromethane, or a combination thereof.

10. The method for preparing finerenone according to claim 7, **characterized in that** in step (2), a weight ratio of palladium on carbon to compound IV of S-configuration is 0.2% to 2.5%:1.
